# EUROPEAN PATENT APPLICATION

(11) **EP 4 317 985 A1**
(43) Date of publication of application: **07.02.2024**
(21) Application number: 22775057.7
(22) Date of filing: 07.03.2022
(51) Int. Cl.: G01N 35/00, G01N 33/543, G01N 21/17, G01N 21/78

(54) **IMMUNOCHROMOTOGRAPHIC INSPECTION APPARATUS**

(30) Priority: 24.03.2021 JP 2021050778
(71) Applicant: FUJIFILM Corporation, Tokyo 106-8620 (JP)
(72) Inventor: TANAKA, Yasutake, Ashigarakami-gun, Kanagawa 258-8538 (JP); ISHII, Hiroyasu, Ashigarakami-gun, Kanagawa 258-8538 (JP)
(74) Representative: Hughes, Andrea Michelle
(86) International application number: PCT/JP2022/009823
(87) International publication number: WO 2022/202263

(57) **Abstract**

An immunochromatographic assay apparatus includes a loading part in which a cartridge that includes a carrier having a spotting region on which a sample is spotted and an assay region in which a color development state changes depending on whether the sample is positive or negative, and in which a reagent is supplied to the carrier, is attachably and detachably loaded, a first detection unit that detects a color development state in the assay region, a second detection unit that detects a generation state of bubbles generated in the assay region due to the supply of the reagent, and a processor configured to discriminate presence or absence of a change in the color development state based on the color development state detected by the first detection unit, and discriminate the presence or absence of the change in the color development state in a case where the bubbles are discriminated to be absent based on the generation state of the bubbles detected by the second detection unit.

## Description

### BACKGROUND OF THE INVENTION

### 1. Field of the Invention

The present disclosure relates to an immunochromatographic assay apparatus.

### 2. Description of the Related Art

Among immunoassay methods, an immunochromatographic method is generally widely utilized, because operation is easy and assay can be performed within a short period of time.

WO2016/114122A and WO2017/104143A disclose an immunochromatographic kit using an immunochromatographic method. The immunochromatographic kit includes an immunochromatographic carrier to which a sample is supplied. The immunochromatographic carrier provides with an assay region on which an antibody that specifically binds to an antigen, which is a test substance, is immobilized. In a case where a labeled antibody that specifically binds to an antigen is developed on an immunochromatographic carrier together with a sample containing the antigen, the antigen binds to the antibody immobilized on the assay region and the labeling substance is captured via the antigen. In a case where the assay region develops a color by the labeling substance captured in the assay region, it is determined that the sample is positive. In a case where the amount of the labeling substance captured in the assay region is very small, the color development is weak and it may be determined that the sample is negative. Therefore, WO2016/114122A and WO2017/104143A disclose an amplification technique for amplifying a labeling signal emitted by a labeling substance. The disclosed amplification technique is a silver amplification technique in which gold colloidal particles are used as a labeling substance and silver ions and a silver ion reducing agent are used as reagents for amplification. In the silver amplification, an amplification reaction is caused in which using the gold colloidal particles as a catalyst, silver particles having a relatively large particle diameter are generated. By this amplification reaction, the labeling signal emitted by the gold colloidal particles is amplified.

The immunochromatographic kits according to WO2016/114122A and WO2017/104143A include a first amplifying liquid pod holding a first amplifying liquid (corresponding to a first reagent) containing a silver ion reducing agent, and a second amplifying liquid that holds a second amplifying liquid (corresponding to a second reagent) containing silver ions. The immunochromatographic kit includes a mechanism for applying a pressing force to the first amplifying liquid pod and a mechanism for applying a pressing force to the second amplifying liquid pod. By applying a pressing force to each mechanism, it is possible to supply the first amplifying liquid and the second amplifying liquid to the immunochromatographic carrier to cause an amplification reaction.

JP2008-275473A relates to an analysis apparatus that performs analysis processing on a large number of samples in parallel by using a microwell provided with a plurality of wells. The analysis apparatus according to JP2008-275473A includes a bubble and foreign substance determination unit for determining the inclusion of bubbles or foreign substances in the microwell. In performing the assay whether the sample in each well is positive or negative, mixing of bubbles or foreign substances in the well may cause an erroneous determination. Therefore, in JP2008-275473A, the mixing of bubbles or foreign substances is determined by the bubble and foreign substance determination unit, and it is determined whether the sample is positive or negative based on the determination result by the bubble and foreign substance determination unit.

### SUMMARY OF THE INVENTION

As the immunochromatographic kits described in WO2016/114122A and WO2017/104143A, an assay apparatus using a cartridge including a first amplifying liquid pod and a second amplifying liquid pod is known. The assay apparatus includes a sensor that optically detects a luminescent state of the assay region and a display unit that displays the detection result. By using the assay apparatus, it is possible for the user to perform determination mechanically whether the sample is a positive or negative only by performing the operation of loading the cartridge in which the sample is spotted.

In the immunochromatographic kit disclosed in WO2016/114122A and WO2017/104143A, it has become clear that bubbles may be generated in an assay region in a case where a second amplifying liquid is developed on a carrier. In a case where the determination of the assay region is performed in a state where bubbles are present in the assay region, an erroneous determination may occur in the determination result.

In the analysis apparatus of JP2008-275473A, in a case where it is determined that bubbles occupy not less than specified region of the determination region, it is undeterminable whether the sample is positive or negative by the bubbles, and thus it is to be processed by visual observation by an operator or to be re-assayed. In this way, in a case where the assay is performed in a state where bubbles are present, it may be undeterminable whether the sample is positive or negative in the analysis apparatus. In the analysis apparatus, it is desired to suppress the occurrence of erroneous determination and to suppress the number of samples that is undeterminable and to be re-assayed.

The present disclosure has been made in view of the above circumstances, and an object of the present disclosure is to provide an immunochromatographic assay apparatus capable of suppressing the occurrence of erroneous determination as compared with the prior art.

The immunochromatographic assay apparatus of the present disclosure includes a loading part in which a cartridge that includes a carrier having a spotting region on which a sample is spotted and an assay region in which a color development state changes depending on whether the sample is positive or negative, and in which a reagent is supplied to the carrier, is attachably and detachably loaded,
a first detection unit that detects a color development state in the assay region,
a second detection unit that detects a generation state of bubbles generated in the assay region due to the supply of the reagent, and
a processor configured to discriminate presence or absence of a change in the color development state based on the color development state detected by the first detection unit, and discriminate the presence or absence of the change in the color development state in a case where the bubbles are discriminated to be absent based on the generation state of bubbles detected by the second detection unit.

In the immunochromatographic assay apparatus of the present disclosure, the second detection unit is preferably an image sensor that images an observation region including the assay region and a peripheral region of the assay region to output an observation image including the observation region.

In the immunochromatographic assay apparatus of the present disclosure, the processor may be configured to obtain a standard deviation of pixel values of an image of a region of interest within the observation region, and discriminate the bubbles to be absent in a case where the standard deviation is not more than a preset threshold value.

In the immunochromatographic assay apparatus of the present disclosure, the processor may be configured to derive a difference or a ratio between a pixel value between a reference image in a state where bubbles are absent and an image of a region of interest within the observation region, and discriminate the bubbles to be absent in a case where the difference or the ratio is not more than a preset threshold value.

In the immunochromatographic assay apparatus of the present disclosure, in a case where it is discriminated that the bubbles are present, the processor is preferably configured to discriminate presence or absence of the bubbles again after a lapse of a preset first setting time.

In the immunochromatographic assay apparatus of the present disclosure, in a case where it is discriminated that the bubbles are present even after a lapse of a preset second setting time from a preset time point after the cartridge is loaded, the processor is preferably configured to notify an error.

In the immunochromatographic assay apparatus of the present disclosure, the processor may be configured to acquire two or more of observation images having a time difference from the second detection unit, derive a difference or a ratio between pixel values, between images of a region of interest within the observation region in the two or more of observation images, and discriminate the bubbles to be absent in a case where the difference or the ratio is not more than a preset threshold value.

In the immunochromatographic assay apparatus of the present disclosure, it is preferable that the first detection unit is an imaging unit that images an observation region including the assay region and a peripheral region thereof, and the first detection unit and the second detection unit are used in common.

In the immunochromatographic assay apparatus of the present disclosure, it is preferable that the cartridge includes a cover member that covers the carrier, and in the cover member, has a gap of 0.01 to 1 mm in which a reagent is supplied between a surface of the carrier in the assay region and the cover member, and the reagent is developed in the assay region using the gap as a flow channel.

In the immunochromatographic assay apparatus of the present disclosure, it is preferable that the cartridge includes a first reagent holding part that holds a first reagent to be supplied to the carrier and a second reagent holding part that holds a second reagent to be supplied to the carrier after the first reagent is supplied to the carrier, the reagent that is a cause for generation of the bubbles is the second reagent, and the processor is configured to discriminate presence or absence of the bubbles after the second reagent is supplied to the carrier.

According to the immunochromatographic assay apparatus of the present disclosure, the occurrence of erroneous determination can be suppressed.

### BRIEF DESCRIPTION OF THE DRAWINGS

Fig. 1 is a perspective view showing appearance of an immunochromatographic assay apparatus.
Fig. 2 is a perspective view of a cartridge.
Fig. 3 is an exploded perspective view of the cartridge.
Fig. 4 is a diagram showing a positional relationship between an assay strip, a multifunctional member, a first reagent holding part, and a second reagent holding part, in the cartridge.
Fig. 5 is an explanatory diagram of an immunochromatographic method.
Fig. 6 is a partially broken side view of an assay apparatus in a state where the cartridge is loaded.
Fig. 7 is a partially broken side view of the assay apparatus in a state where the cartridge is loaded and a second reagent supply mechanism is operated.
Fig. 8A and Fig. 8B are diagrams schematically showing an image of an imaging region, where Fig. 8A is a diagram showing a case where bubbles are present and Fig. 8B is a diagram showing a case where bubbles are absent.
Fig. 9 is a diagram showing an assay flow.
Fig. 10 is a diagram showing an assay flow in an assay apparatus.
Fig. 11 is a diagram showing a first flow of a step of discriminating presence or absence of bubbles.
Fig. 12 is a diagram showing a second flow of a step of discriminating presence or absence of bubbles.
Fig. 13 is a diagram showing a modified example of an assay flow in the assay apparatus.
Fig. 14 is a diagram showing a flow of a step of confirming that bubbles are absent.

### DESCRIPTION OF THE PREFERRED EMBODIMENTS

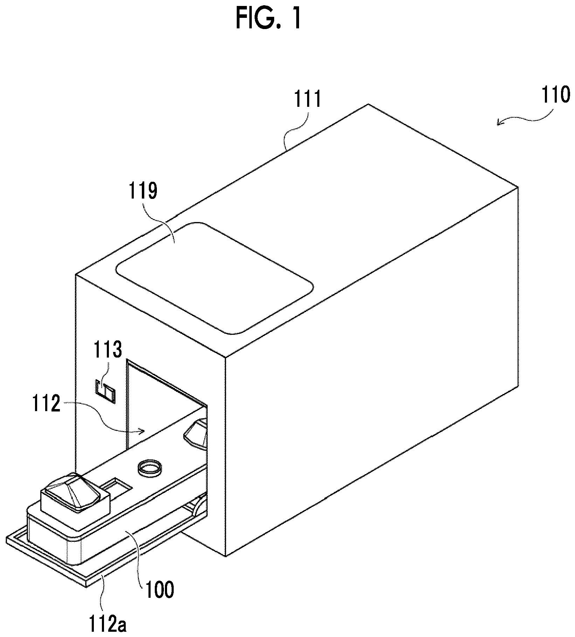

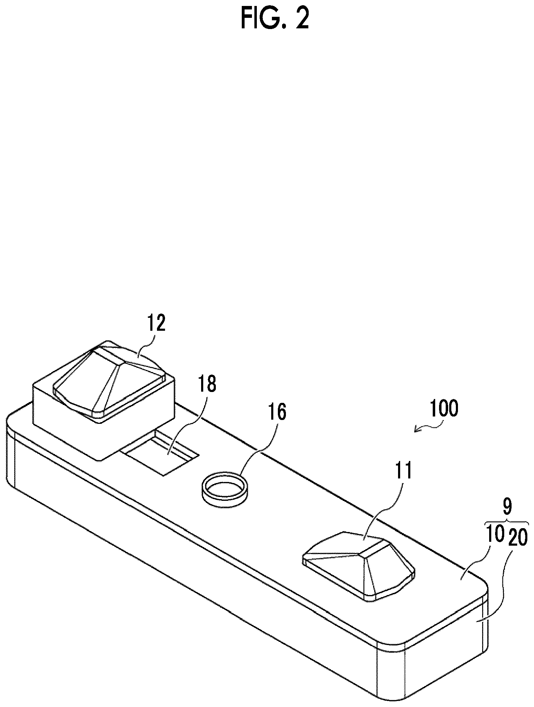

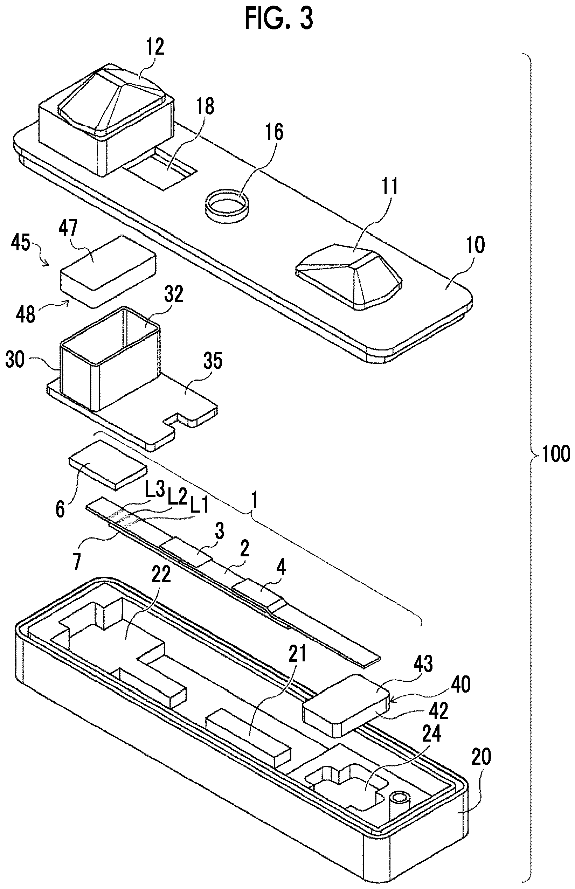

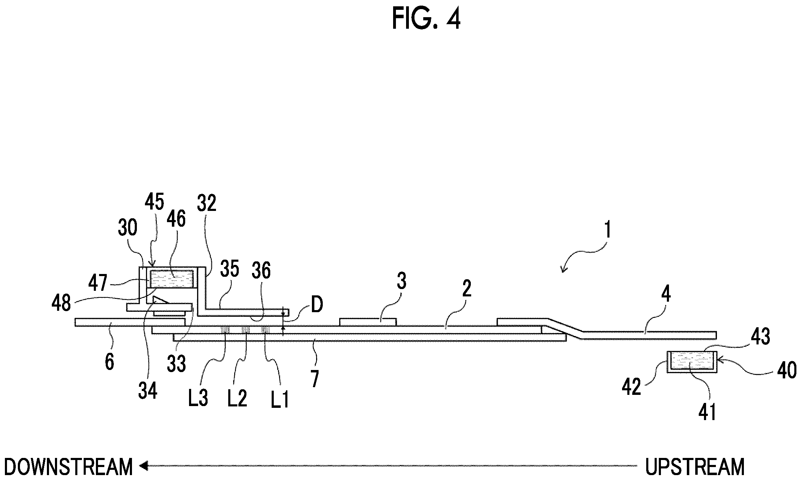

Embodiments of the immunochromatographic assay apparatus of the present disclosure will be described with reference to the drawings. Fig. 1 is a perspective view illustrating the appearance of the immunochromatographic assay apparatus 110 (hereinafter, simply referred to as an assay apparatus 110) according to one embodiment. Fig. 2 is an external view of a cartridge 100 mounted on the assay apparatus 110 and Fig. 3 is an exploded perspective view of the cartridge 100. Fig. 4 is a diagram illustrating the positional relationship of the main accommodated components in the cartridge 100.

The cartridge 100 is a single-use type that is used one by one for each sample of assay target. As shown in Fig. 3, an assay strip 1 including an immunochromatographic carrier 2 (hereinafter, referred to as a carrier 2) is disposed in the cartridge 100. An assay region L1 is provided in the carrier 2, and the color development state changes depending on whether or not the sample contains a test substance, that is, whether the sample is positive or negative.

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, a liquid specimen containing animals and plants themselves or a dried body thereof. Examples of the test substance include an antigen, an antibody, a protein, and a low-molecular-weight compound.

In the assay apparatus 110 of the present example, the cartridge 100 in a state in which the sample is spotted is loaded. Then, the assay apparatus 110 detects a color development state of the assay region L1 of the loaded cartridge 100, and presents the result of whether the sample is positive or negative. Hereinafter, a determination as to whether the sample is positive or negative will be referred to as a main determination. In a case of where a plurality of samples are assayed, the cartridge 100 for each sample is loaded one by one into the assay apparatus 110.

Hereinafter, the cartridge 100 will be described on the premise that the cartridge 100 is loaded into the assay apparatus 110. However, the cartridge 100 of the present example has a configuration that a user can confirm visually whether the sample is positive or negative without using the assay apparatus 110. Such a cartridge 100 is also referred to as an immunochromatographic assay tool, an immunochromatographic assay kit, or the like.

As shown in Fig. 1, the assay apparatus 110 includes a case body 111, and the case body 111 includes a cartridge loading part 112 in which the cartridge 100 is attachably and detachably loaded. As an example, an opening for inserting the cartridge 100 into the case body 111 and an opening/closing lid 112a for opening and closing the opening are provided on the front surface of the case body 111. The opening/closing lid 112a is opened when the cartridge 100 is loaded, the cartridge 100 is inserted into the case body 111, and the opening/closing lid 112a is closed after the cartridge 100 has been loaded into the cartridge loading part 112. The assay is performed in a state where the opening/closing lid 112a is closed.

In addition, a power switch 113 is provided on the front surface of the case body 111, and a monitor 119 is provided on the upper surface of the case body 111. A result of the main determination, an error message, and the like are displayed on the monitor 119. As an example, the monitor 119 is a touch panel monitor, and various operation screens are displayed. Through the operation screen, the user can input a start instruction of processing and an operation instruction such as selection of an assay procedure.

As shown in Fig. 2 and Fig. 3, as an example, the cartridge 100 includes a housing 9 constituted of a case member 20 and a cover member 10. The housing 9 is formed of, for example, a resin material. An opening is formed in an upper part of the case member 20, and in addition to the assay strip 1, a first reagent holding part 40, a second reagent holding part 45, and the like are accommodated therein. The cover member 10 covers the opening of the case member 20 by being attached to the opening part of the case member 20. The housing 9 has an elongated shape as a whole in accordance with the elongated shape of the assay strip 1.

In the present example, a dropping port 16, an observation window 18, a first pressed part 11, and a second pressed part 12 are provided on an upper part of the housing 9 constituted of the cover member 10. Each of these parts is integrally molded with the cover member 10 as an example. The dropping port 16 is an opening for adding dropwise a sample into the inside of the housing 9. A boss is vertically provided on the edge of the dropping port 16 toward the upper part. The observation window 18 is a window for observing the assay region L1 from the outside, and is formed of a transparent member as an example. In the present example, the size of the observation window 18 is a size such that, in addition to the assay region L1, the control region L2 and the color development region L3, which will be described later, can also be observed.

The first pressed part 11 is an operating part operated to supply the first reagent 41 (see Fig. 4) in the first reagent holding part 40 to the carrier 2. The second pressed part 12 is an operating part operated to supply the second reagent 46 (see Fig. 4) in the second reagent holding part 45 to the carrier 2. As will be described later, the first reagent 41 and the second reagent 46 are amplification agents for amplifying the color development in the assay region L1 in a case where a sample 50 is positive.

In a case where a pressing force is applied from the outside as an external force to the first pressed part 11, the first pressed part 11 is deformed. As an example, the first pressed part 11 has a quadrangular pyramid shape, and in a case where a pressing force is applied from above to a region including the apex of the quadrangular pyramid, the first pressed part 11 is deformed such that the apex of the quadrangular pyramid sinks into the inside of the housing 9. In a case of where the first pressed part 11 is deformed in this manner, a pressing force is applied to the first reagent holding part 40 inside the housing 9. In the first reagent holding part 40, deformation or the like due to a pressing force applied through the first pressed part 11 occurs. Due to this deformation or the like, the first reagent 41 held by the first reagent holding part 40 is supplied to the assay strip 1.

In addition, it is preferable that the first pressed part 11 is deformed by pressing and then the deformed state is maintained. The reason is as follows. As will be described later, in the assay apparatus 110 of the present example, the cartridge 100 in a state in which the first pressed part 11 is pressed in advance by the user can be loaded. It is because, in a case where the first pressed part 11 is pressed by the user before being loaded into the assay apparatus 110, the first pressed part 11 in which the deformation is maintained even after the user releases the hand, is easier to continue the supply of the first reagent 41.

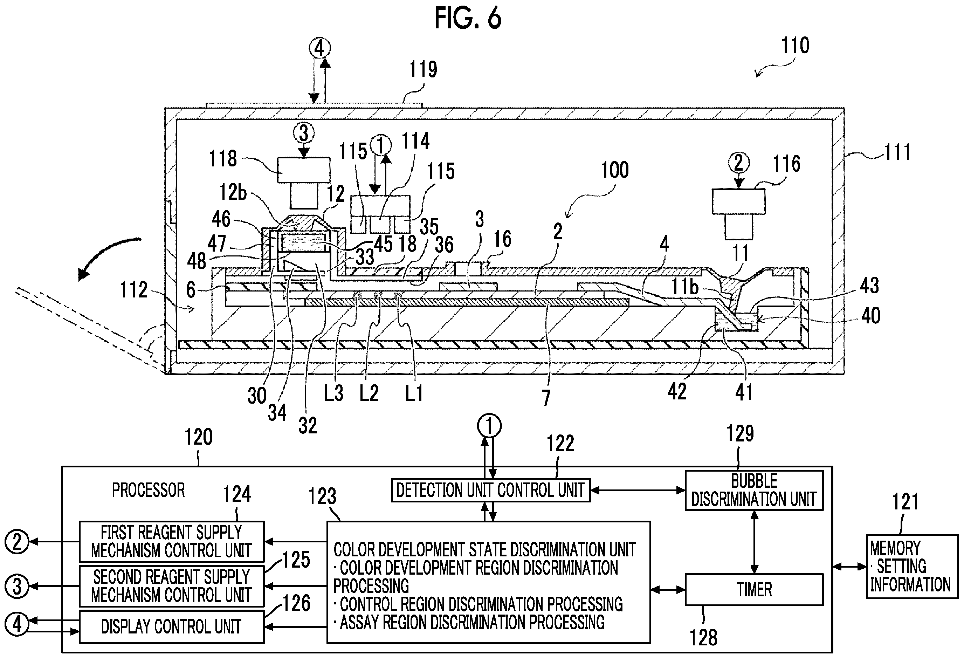

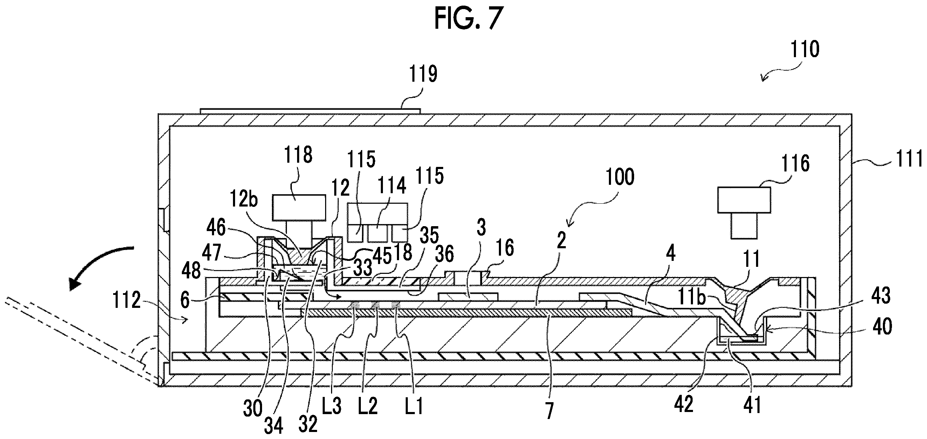

Similarly, in a case where a pressing force is applied from the outside as an external force to the second pressed part 12, the second pressed part 12 is deformed. Similarly to the first pressed part 11, the second pressed part 12 of the present example also has a quadrangular pyramid shape, and in a case where a pressing force is applied from above to a region including the apex of the quadrangular pyramid, the second pressed part 12 is deformed such that the apex of the quadrangular pyramid sinks into the inside of the housing 9. In a case of where the second pressed part 12 is deformed in this manner, a pressing force is applied to the second reagent holding part 45 inside the housing 9. In the second reagent holding part 45, deformation or the like due to a pressing force applied through the second pressed part 12 occurs. Due to this deformation or the like, the second reagent 46 held by the second reagent holding part 45 is supplied to the assay strip 1. In the second pressed part 12 of the present example, an abutting part 12b that abuts on the second reagent holding part 45 is provided (see Fig. 6 and Fig. 7).

As will be described later, in the assay apparatus 110 of the present example, a plurality of assay flows can be selected. In a case where in the assay apparatus 110, the assay flow for supplying the second reagent 46 is selected from the assay flows that can be selected in the assay apparatus 110, the second pressed part 12 is pressed by the internal mechanism of the assay apparatus 110. Therefore, the second pressed part 12 may be depressible by an internal mechanism. In a case of selecting an assay flow in which the cartridge 100 is loaded into the assay apparatus 110 after the supply of the first reagent 41 and the second reagent 46, a case where the cartridge 100 is used is preferably an aspect that the second pressed part 12 is also depressible by the user.

As shown in Fig. 3 and Fig. 4, the case member 20 accommodates the assay strip 1 including the carrier 2 along the longitudinal direction. In the case member 20, the first reagent holding part 40 is disposed on one end part side (upstream side shown in Fig. 4) in the longitudinal direction. In the case member 20, in a portion where the first reagent holding part 40 is disposed, the first accommodating part 24 that is a recess-shaped in accordance with the shape of the first reagent holding part 40 is formed. One end part of the assay strip 1 is disposed above the first reagent holding part 40 in a state of being accommodated in the first accommodating part 24.

The first reagent holding part 40 holds the first reagent 41. The first reagent holding part 40 is constituted of, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward. The pressing force applied from the first pressed part 11 is transmitted to the sheet member 43 of the first reagent holding part 40 via the end part of the assay strip 1 to break the sheet member 43 (see Fig. 6 and Fig. 7). The sheet member 43 is broken, and thus the first reagent 41 is supplied to the assay strip 1. In the first pressed part 11 of the present example, a protruding part 11b that abuts on the sheet member 43 (see Fig. 6 and Fig. 7). The protruding part 11b has, for example, an elongated shape extending in the longitudinal direction in the width direction of the assay strip 1 and a pointed shape toward the sheet member 43, such that the sheet member 43 is easily broken.

In addition, the cartridge 100 includes a multifunctional member 30 having a function of accommodating the second reagent holding part 45. The multifunctional member 30 is disposed on the other end part side (downstream side shown in Fig. 4) of the case member 20 and above the assay strip 1. The multifunctional member 30 is a member in which the second accommodating part 32 and the flow channel forming part 35 are integrally formed. The second accommodating part 32 is a part accommodating the second reagent holding part 45. The second accommodating part 32 has a box shape having an opened upper surface. As shown in Fig. 4, on the bottom of the second accommodating part 32, a protrusion 34 for breaking a sheet member 48, which will be described later, of the second reagent holding part 45, and an opening 33 that allows to flow the second reagent 46 flowed out from the second reagent holding part 45, toward the carrier 2.

The flow channel forming part 35 is provided to be connected to the upstream side from the second accommodating part 32. The flow channel forming part 35 has a flat plate shape, is disposed at a position facing the assay region L1 or the like in the longitudinal direction of the carrier 2, and is disposed with an interval from the carrier 2. Then, between the flow channel forming part 35 and the carrier 2, a flow channel for flowing the second reagent 46 flowed out from the second accommodating part 32 toward the assay region L1 or the like is formed. In this way, the flow channel forming part 35 is disposed between the observation window 18 and the assay region L1 or the like of the carrier 2. Therefore, the flow channel forming part 35 is formed of a transparent member and thus the assay region L1 and the like can be observed through the observation window 18.

The second reagent holding part 45 holds the second reagent 46. The second reagent holding part 45 is constituted of, for example, a container 47 formed of a resin material and having an opening on one surface, and a sheet member 48 that covers the opening of the container 47 and is breakable. The container 47 is filled with the second reagent 46, and the opening of the container 47 is sealed by the sheet member 48. The second reagent holding part 45 is disposed in the second accommodating part 32 in a posture in which the sheet member 48 faces downward. Accordingly, the sheet member 48 faces the protrusion 34 in the second accommodating part 32.

The pressing force applied from the second pressed part 12 to the second reagent holding part 45 acts in a direction of pushing down the second reagent holding part 45 downwardly, whereby the sheet member 48 is pressed against the protrusion 34. The sheet member 48 is pressed against the protrusion 34, whereby the sheet member 48 is broken (see Fig. 6 and Fig. 7). The sheet member 48 is broken, and thus the second reagent 46 is supplied to the carrier 2 through the flow channel formed by the opening 33 at the bottom of the second accommodating part 32 and the flow channel forming part 35.

As shown in Fig. 4, a gap (a clearance) D corresponding to the flow channel for the second reagent 46 is formed between a back surface 36 of the flow channel forming part 35 of the multifunctional member 30 and the carrier 2 of the assay strip 1. The gap D is, for example, in the range of 0.01 mm to 1 mm. The second reagent 46 flows out from the opening 33 at the bottom of the second accommodating part 32 toward the carrier 2, and the second reagent 46 that has flowed out flows through the flow channel formed by the gap D and reaches at least above the assay region L1. The second reagent 46 that has reached the assay region L1 infiltrates the assay region L1 from the flow channel. That is, in the housing 9, a gap D of 0.01 to 1 mm in which the second reagent 46 is supplied is provided between the surface of the carrier 2 in the assay region L1 and the flow channel forming part 35, and the second reagent 46 is developed in the assay region L1 using the gap D as a flow channel.

An absorption pad 6, which will be described later, is disposed at an end part on the downstream side of the assay strip 1. In the case member 20, a support part 22 that supports an end part of the assay strip 1 including the absorption pad 6 is formed at a position facing the absorption pad 6. A second accommodating part 32 of the multifunctional member 30 is disposed above the absorption pad 6. The support part 22 also supports the multifunctional member 30 via the absorption pad 6. In addition, in the case member 20, a support part 21 that supports a central part of the assay strip 1 is formed.

The assay strip 1 includes a carrier 2, a liquid feeding pad 4, and an absorption pad 6. Then, the carrier 2 is fixedly supported on a back pressure-sensitive adhesive sheet 7.

The carrier 2 is a porous insoluble carrier for developing a sample, and includes an assay region L1, a control region L2, and a color development region L3. In addition, the carrier 2 includes a label holding pad 3. The label holding pad 3 constitutes a spotting region on which the sample is spotted. The color development region L3 is disposed on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

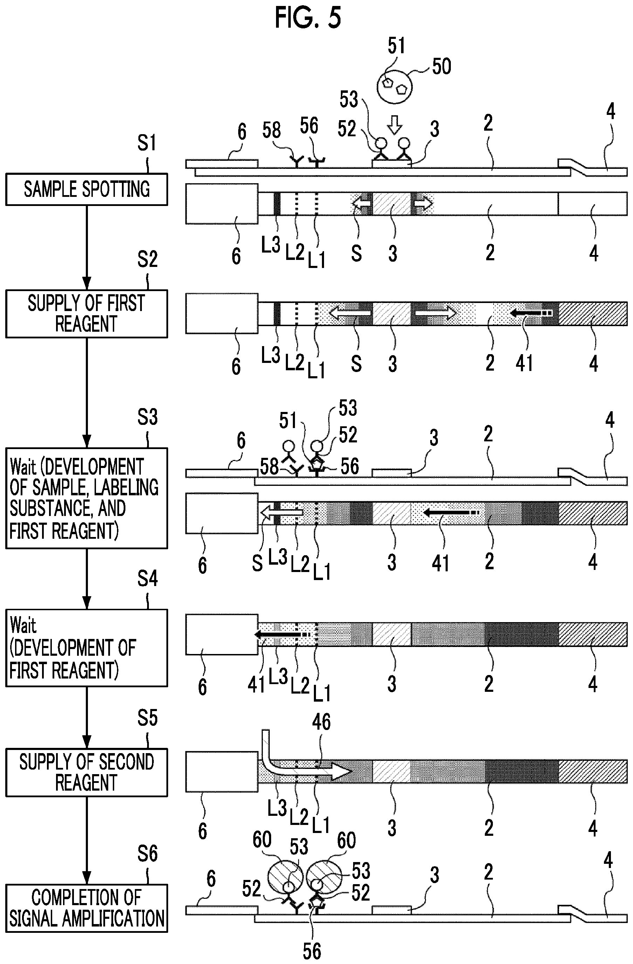

It shows a state in which the assay region L1, the control region L2, and the color development region L3 are expressed as lines, but these are not always expressed. Details will be described later, but before developing the sample 50 (see Fig. 5), the first reagent 41 (see Fig. 4), and the second reagent 46 (see Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. As a result, the assay region L1 becomes visible. Since the color development of the assay region L1 is amplified by silver amplification, which will be described later, the assay region L1 develops a black color.

The control region L2 is also expressed as a line by increasing the color optical density in a case where the sample 50 is developed. As a result, the control region L2 becomes visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

As shown in Fig. 5, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

The first binding substance 52 that modifies the labeling substance 53 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 56 that is fixed in the assay region L1 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFII,M Wako Pure Chemical Corporation) can be used as the third binding substance 58.

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, 038-06925) is used as the first reagent 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

The liquid feeding pad 4 is disposed in contact with one end of the carrier 2 and the first reagent 41 is fed to the carrier 2 from the upstream side of the spotting region (constituted of the label holding pad 3). In the liquid feeding pad 4, in a case where the first pressed part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary phenomenon.

The absorption pad 6 is disposed in contact with the other end of the carrier 2 and absorbs the sample 50, the first reagent 41, and the second reagent 46, which are developed on the carrier 2. The absorption pad 6 is also formed of a porous material.

In the present embodiment, the first reagent 41 and the second reagent 46 are amplification agents that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplification agents used for silver amplification, and the reaction between the first reagent 41 and the second reagent 46 using the labeling substance 53 as a catalyst is an amplification reaction. By the amplification reaction, silver particles 60 (see Fig. 5) having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, silver particles 60 (see Fig. 5) are generated, and the generated silver particles 60 deposits on the labeling substance 53 using the labeling substance 53 as a nucleus. By depositing the silver particles on the labeling substance 53, silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

### (First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. In a case where an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of Fe²⁺ is preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic method>

An immunochromatographic method will be described with reference to Fig. 5. Here, a case where the sample 50 includes the test substance 51, that is, on the premise that the sample 50 is positive will be described.

First, the sample 50 is spotted on the label holding pad 3 which is the spotting region (Step S1). The test substance 51 in the sample 50, which is spotted on the label holding pad 3, specifically binds to the first binding substance 52 that modifies the labeling substance 53 contained in the label holding pad 3. The sample 50 is developed on the downstream side from the label holding pad 3 in the carrier 2 by the capillary phenomenon in the carrier 2. A part of the sample 50 is also developed on the upstream side. The arrow S indicates a state in which the sample 50 is developed.

Next, the first reagent 41 is supplied (Step S2). The first reagent 41 is supplied from the liquid feeding pad 4. The first reagent 41 is supplied to the carrier 2 via the liquid feeding pad 4 and is developed on the downstream side.

After that, the process waits until the first reagent 41 is developed on the downstream side (Step S3 and Step S4). "Wait" shown in Fig. 5 means an action of waiting. The first reagent 41 is gradually developed to the downstream side, and the sample 50 to be developed from the label holding pad 3 and the labeling substance 53 modified with the first binding substance 52 are developed to the downstream side to be pushed by the first reagent 41 (Step S3).

The test substance 51 in the sample 50 that has been developed to the downstream side and has reached the assay region L1 is captured by the second binding substance 56 of the assay region L1. That is, the labeling substance 53 is captured in the assay region L1 via the test substance 51 and the first binding substance 52. On the other hand, the labeling substance 53 that is not bound to the test substance 51 passes through the assay region L1 without being captured and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed on the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

In the cartridge 100 according to the present embodiment, the first reagent 41 is developed on the downstream side due to the capillary action in the liquid feeding pad 4 and the carrier 2. On the other hand, the second reagent 46 flows on the surface of the carrier 2 using the gap D between the flow channel forming part 35 of the multifunctional member 30 and the surface of the carrier 2 as a flow channel and is developed in the assay region L1. In a case where the second reagent 46 flows out from the opening 33 at the bottom of the second accommodating part 32 of the multifunctional member 30 into the gap D serving as a flow channel and in a case where the second reagent 46 flows through the gap D, bubbles may be generated in the gap D. That is, in a case where the second reagent 46 is supplied to the carrier 2, bubbles may be generated on the assay region L1 arranged at a position facing the flow channel forming part 35 of the carrier 2 and the periphery of the assay region L1. It is presumed that one of the causes of the generation of bubbles is that the second reagent 46 flows vigorously into the gap D and the accumulated air and the second reagent 46 are mixed with each other. In addition, it is also presumed that the cause is that, in a case where the second reagent 46 that has flowed into the gap D infiltrates into the carrier 2, the air in the carrier 2 floats up on the surface of the carrier 2. It is presumed that bubbles are generated due to at least one of these.

Fig. 6 and Fig. 7 are partially broken side views of the assay apparatus 110 in a state where the cartridge 100 is loaded. Hereinafter, a configuration and a function of the assay apparatus 110 will be described with reference to Fig. 6 and Fig. 7.

In the assay apparatus 110 of the present example, for example, the following three assay flows of a first assay flow, a second assay flow, and a third assay flow can be selected. In any of the first to third assay flows, it is necessary that the sample 50 is spotted on the carrier 2 of the cartridge 100 before loading.

The first assay flow is a flow in which an assay is performed on the cartridge 100 in a state where the spotting of the sample 50 and the supply of the first reagent 41 are started before loading. In the case of the first assay flow, after the cartridge is loaded into the assay apparatus 110, only the supply of the second reagent 46 to the carrier 2, among the first reagent 41 and the second reagent 46, is performed by the assay apparatus 110.

The second assay flow is a flow in which an assay is performed on the cartridge 100 in which only the spotting of the sample 50 is performed before loading. In the case of the second assay flow, after the cartridge is loaded into the assay apparatus 110, the supply of both the first reagent 41 and the second reagent 46 to the carrier 2 is performed by the assay apparatus 110.

The third assay flow is a flow in which an assay is performed on the cartridge 100 in a state where the spotting of the sample 50, the supply of the first reagent 41, and the supply of the second reagent 46 are started before loading. In the case of the third assay flow, after the cartridge is loaded into the assay apparatus 110, the supply of the first reagent 41 and the second reagent 46 is not performed in the assay apparatus 110.

Hereinafter, a configuration of the assay apparatus 110 will be described, and then a first assay flow will be described.

### (Configuration of assay apparatus 110)

As shown in Fig. 6 and Fig. 7, the assay apparatus 110 includes a first reagent supply mechanism 116 and a second reagent supply mechanism 118 as internal mechanisms. The first reagent supply mechanism 116 is a mechanism for starting the supply of the first reagent 41 from the first reagent holding part 40 to the carrier 2. As the first reagent supply mechanism 116, for example, an actuator such as a solenoid provided with an electromagnet and a plunger movable with respect to the electromagnet is used. For example, as the plunger moves, the plunger abuts on the first pressed part 11 and presses the first pressed part 11. The first reagent supply mechanism 116 is disposed at a position facing the first pressed part 11 of the loaded cartridge 100.

The first reagent supply mechanism 116 is a pressing mechanism that applies a pressing force to the first pressed part 11 from the outside by pressing the first pressed part 11 of the cartridge 100. In a case where a pressing force is applied to the first pressed part 11 by the first reagent supply mechanism 116, the first reagent 41 is supplied from the first reagent holding part 40 to the carrier 2 by the above-described action. In the first assay flow and the third assay flow, the first reagent supply mechanism 116 is not used and only in the second assay flow, the first reagent supply mechanism 116 is used.

The second reagent supply mechanism 118 is a mechanism for starting the supply of the second reagent 46 from the second reagent holding part 45 to the carrier 2. Also as the second reagent supply mechanism 118, an actuator such as a solenoid is used similarity to the first reagent supply mechanism 116. The second reagent supply mechanism 118 is disposed at a position facing the second pressed part 12 of the loaded cartridge 100. The second reagent supply mechanism 118 is a pressing mechanism that applies a pressing force to the second pressed part 12 from the outside by pressing the second pressed part 12 of the cartridge 100. In a case where a pressing force is applied to the second pressed part 12 by the second reagent supply mechanism 118, the second reagent 46 is supplied from the second reagent holding part 45 to the carrier 2 by the above-described action. In the third assay flow, the second reagent supply mechanism 118 is not used and only in the first assay flow and the second assay flow, the second reagent supply mechanism 118 is used.

In addition to the loading part 112, the first reagent supply mechanism 116, and the second reagent supply mechanism 118, the assay apparatus 110 further includes a detection unit 114, a processor 120, and a memory 121 in the case body 111. In Fig. 6, the processor 120 and the memory 121 are illustrated outside the case body 111 of the assay apparatus 110, but this is a schematic diagram and the processor 120 and the memory 121 are actually disposed inside the case body 111.

The detection unit 114 optically detects the color development state of the assay region L1, the control region L2, and the color development region L3, and outputs a detection signal indicating the color development state to the processor 120. In addition, the detection unit 114 detects a generation state of the bubbles generated in the assay region L1 due to the supply of the second reagent 46, and outputs a detection signal indicating the generation state of bubbles to the processor 120.

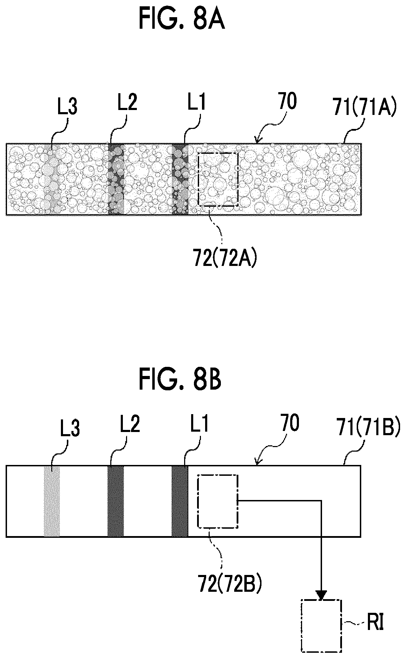

The detection unit 114 is an image sensor such as a complementary metal oxide semiconductor (CMOS) image sensor and a charge coupled device (CCD) image sensor. The detection unit 114 is disposed, for example, at a position facing the observation window 18. The detection unit 114 images a preset range including the observation window 18 as the center and the periphery thereof. The captured image imaged by the detection unit 114 includes an observation region 70 (see Fig. 8) exposed from the observation window 18, such as an assay region L1, a control region L2, and a color development region L3. Then, the captured image is output from the detection unit 114 to the processor 120.

In addition, as an example, a light source 115 such as a light emitting diode that illuminates the assay region L1, the control region L2, and the color development region L3 during the imaging is provided on both sides of the detection unit 114.

The processor 120 integrally controls each part of the assay apparatus 110. An example of the processor 120 is a Central Processing Unit (CPU) that performs various types of control by executing a program. The CPU functions as a control unit having a detection unit control unit 122, a color development state discrimination unit 123, a first reagent supply mechanism control unit 124, a second reagent supply mechanism control unit 125, a display control unit 126, a bubble discrimination unit 129, and a timer 128 by executing a program. The memory 121 is an example of a memory connected to or built in the CPU as the processor 120. For example, a control program is stored in the memory 121. The processor 120 is realized by the CPU executing a control program.

The detection unit control unit 122 controls the imaging timing by the detection unit 114.

The first reagent supply mechanism control unit 124 operates the first reagent supply mechanism 116 to control the first pressed part 11 to be pressed.

The second reagent supply mechanism control unit 125 operates the second reagent supply mechanism 118 based on the change in the color development state of the color development region L3 to control the second pressed part 12 to be pressed.

The color development state discrimination unit 123 executes the color development region discrimination processing, the control region discrimination processing, and the assay region discrimination processing based on the detection signal output by the detection unit 114. As described above, the detection unit 114 outputs the captured image of the observation region 70 including the assay region L1, the control region L2, and the color development region L3. The color development state discrimination unit 123 executes each of the above-described discrimination processing based on the captured image.

The color development region discrimination processing is a processing of discriminating, based on the captured image, a change in the color development state of the color development region L3, as an example, whether or not the color has changed from a dark green color, which is the color before the reaction with the first reagent 41, to an orange color. "Presence" of the change in the color development state means that the first reagent 41 is developed to the color development region L3.

The "change in color development state" includes any of an aspect in which a first color different from the color of the carrier changes to another second color (that is, a color change), an aspect in which the color of the carrier changes to another color by developing a color different from that of the carrier (that is, color development), and an aspect in which the density of the color changes (that is, a change in density).

The processor 120 operates the second reagent supply mechanism 118 via the second reagent supply mechanism control unit 125 in a case where the color development state discrimination unit 123 discriminates that the color development state in the color development region L3 has changed.

The control region discrimination processing is a processing of discriminating presence or absence of a change in the color development state of the control region L2 based on the captured image. In the present example, since a line is expressed in the control region L2 by the labeling substance 53 being captured in the control region, or the silver amplification after capturing the labeling substance 53, presence or absence of the expression of the line in the control region L2 is discriminated. In a case where it is discriminated that the color development state of the control region L2 is changed, that is, the expression in the control region L2 is present, the color development state discrimination unit 123 executes the assay region discrimination processing of the next step.

The assay region discrimination processing is a processing of discriminating presence or absence of a change in the color development state of the assay region L1 based on the captured image. In the present example, since a line is expressed in the assay region L1 by the labeling substance 53 being captured in the assay region L1, or the silver amplification after capturing the labeling substance 53, presence or absence of the expression of the line in the assay region L1 is determined.

In a case where the color development state discrimination unit 123 discriminates that the change in the color development state in the assay region L1 is present, the processor 120 displays the assay result as "positive" on the monitor 119 via the display control unit 126. In addition, in a case where it is discriminated that the change in the color development state in the assay region L1 is absent, the assay result as "negative" is displayed on the monitor 119 via the display control unit 126.

The bubble discrimination unit 129 discriminates the presence or absence of bubbles based on the generation state of bubbles detected by the detection unit 114. The discrimination of the presence or absence of bubbles by the bubble discrimination unit 129 is performed after the operation of the second reagent supply mechanism 118 and before the control region discrimination processing. In a case where the bubble discrimination unit 129 discriminates that the bubbles to be absent, the control region discrimination processing by the color development state discrimination unit 123 is executed.

Fig. 8A and Fig. 8B schematically show an example of the observation image 71 cut out from the captured image output by the detection unit 114. The observation image 71 is an image corresponding to the observation region 70 exposed from the observation window 18 in the carrier 2, and the observation region 70 includes an assay region L1, a control region L2, and a color development region L3.

Both the observation image 71A shown in Fig. 8A and the observation image 71B shown in Fig. 8B are observation images 71 of the observation region 70, and whether or not bubbles are generated in the assay region L1 is different. That is, the observation image 71A shown in Fig. 8A shows a state in which bubbles are generated in the assay region L1, and the observation image 71B shown in Fig. 8B shows a state in which bubbles are not generated in the assay region L1. Hereafter, in a case where it is necessary to distinguish these two images, a subdivided reference numeral of A or B is added to a reference numeral of the observation image 71, and in a case where it is not necessary to distinguish these, the reference numerals are simply referred to as the observation image 71.

As shown in Fig. 8A and Fig. 8B, a state is different between a case where bubbles are generated in the assay region L1 and a case where bubbles are absent, and the difference appears in the observation image 71A and the observation image 71B. In this way, since the observation image 71A in a case where bubbles are present is different from the observation image 71B in a case where bubbles are absent, the bubble discrimination unit 129 discriminates the presence or absence of bubbles in assay region L1 based on the observation image 71 cut out from the captured image. A method of discriminating the presence or absence of bubbles will be described later. In the present example, as shown in the observation image 71A shown in Fig. 8A and the observation image 71B shown in Fig. 8B, a region of interest (ROI) is defined in a peripheral region of the assay region L1, and based on the ROI image 72 corresponding to the defined region of interest ROI, the presence or absence of bubbles is discriminated. Here, the peripheral region of the assay region L1 is a region in which, in a case where bubbles are generated in the assay region L1, bubbles are generated similarly as in the assay region L1, and is a region not including the binding substance for capturing the labeling substance. In a range of, for example, within 5 mm from the assay region L1, a state of presence or absence of bubbles is exhibited according to a state of presence or absence of bubbles in the assay region L1, and thus it can be regarded as a peripheral region in which the region of interest ROI is defined. However, the peripheral region and the region of interest ROI defined in the peripheral region are regions in which the line is not expressed, unlike the assay region L1 and the like. Since the density in the region of interest ROI does not significantly change due to factors other than bubbles regardless of whether the sample 50 is positive or negative, by comparing the ROI image 72A in the image with bubbles such as the observation image 71A and the ROI image 72B in the image without bubbles such as the observation image 71B, the presence or absence of bubbles can be easily discriminated. Also for the ROI image 72, similarly to the observation image 71, in a case where it is necessary to distinguish between the presence and absence of bubbles, a subdivided reference numeral of A or B is added to a reference numeral of the ROI image 72, and in a case where it is not necessary to distinguish these, the reference numerals are simply referred to as ROI image 72.

The ROI image 72B shown in Fig. 8B is a part of the observation image 71B which is an image without bubbles, and the ROI image 72B is used as a reference image RI referred to as a reference for comparison for discriminating the presence or absence of bubbles. The reference image RI in a state where bubbles are absent is held in advance in the memory 121 as, for example, one of the setting information which will be described later.

In addition to the control program, the memory 121 also stores setting information that is preset in order for the processor 120 to perform various types of control. As the setting information, information necessary for the color development state discrimination unit 123 to discriminate a change in the color development state is recorded. Examples of the setting information include a first setting time t1 that is preset, a second setting time t2 that is preset, a third setting time t3 that is preset, a fourth setting time t4 that is preset, and a number of times K and L that is preset, which will be described later. The first setting time t1 is a waiting time, in a case where the processor 120 determines that the bubble is present, until the processor 120 determines again the presence or absence of bubbles. The second setting time t2 is an allowable time in a case where the processor 120 repeatedly discriminates presence or absence of bubbles again in a case where the processor 120 determines that bubbles are present. In addition, the third setting time t3 is a waiting time until the processor 120 determines the presence or absence of a change in the color development state of the color development region L3 again in a case where the processor 120 has been determined that a change in the color development state of the color development region L3 is absent. The fourth setting time t4 is a preset time from a preset time point after the cartridge is loaded, and is an allowable time in a case where the presence or absence of the color development state of the color development region L3 is repeated.

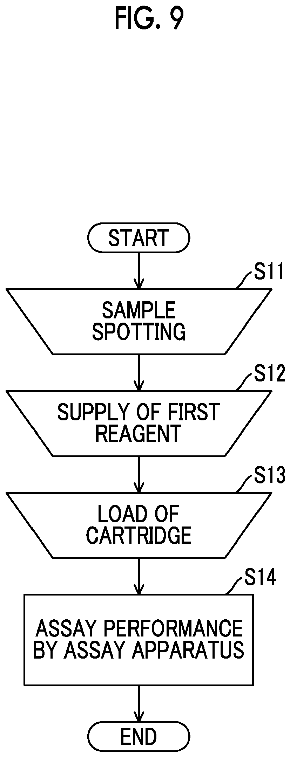

The procedure of the immunochromatographic assay using the assay apparatus 110 of the present embodiment will be described with reference to Fig. 9 to Fig. 11. Here, a first assay flow that is an aspect in which the supply of the first reagent 41 is performed by the user and the supply of the second reagent 46 is performed by the assay apparatus 110 will be described.

### (First assay flow)

Fig. 9 is a diagram showing the first assay flow.

First, a user adds dropwise the sample 50 from the dropping port 16 of the cartridge 100 onto the spotting region of the carrier 2 (Step S11).

Next, the user presses the first pressed part 11 of the cartridge 100 to start the supply of the first reagent 41 (Step S12).

After that, the user loads the cartridge 100 into the loading part 112 of the assay apparatus 110 in a state where a power is turned on (Step S13).

An assay of the loaded cartridge 100 is performed in the assay apparatus 110 (Step S14).

The time from the start of the supply of the first reagent 41 until the first reagent 41 is sufficiently developed in the carrier 2 varies depending on each cartridge, but it takes generally about 5 minutes to 10 minutes. The time from the pressing the first pressed part 11 by the user until the first pressed part 11 is loaded into the loading part 112 may be set according to the convenience of the user.

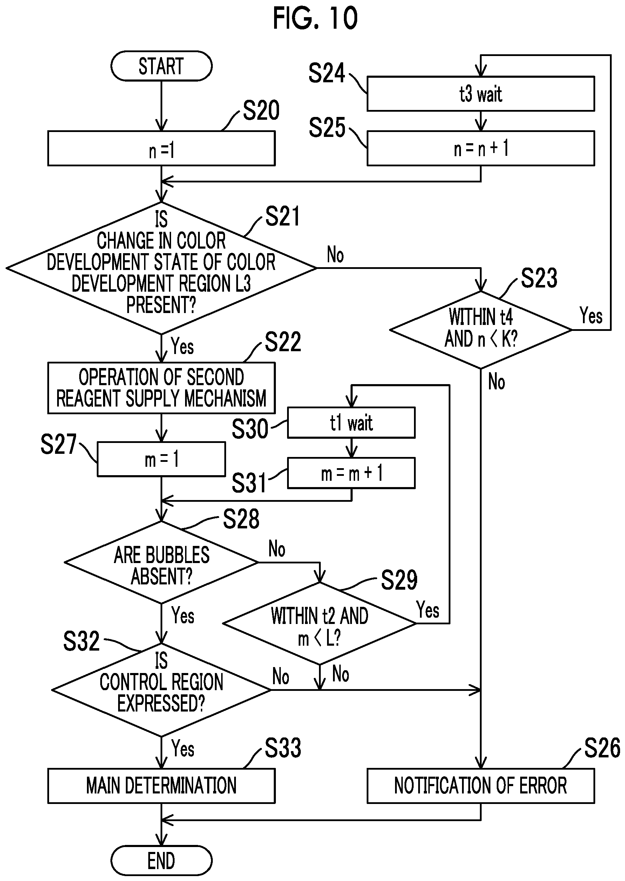

Fig. 10 shows a detailed assay flow of an assay performance (Step S14) by the assay apparatus 110 shown in Fig. 9.

By loading the cartridge 100 into the assay apparatus 110, the assay in the assay apparatus 110 (Step S14 in Fig. 9) is started.

As shown in Fig. 10, in the assay apparatus 110, first, n = 1 is set in the processor 120 (Step S20). Here, n is a parameter of the number of times of executing the discrimination processing of the color development region L3. The processor 120 discriminates whether or not the color development state of the color development region L3 is changed (specifically, the change from a dark green color to an orange color) (Step S21). Specifically, the processor 120 illuminates the observation region 70 exposed from the observation window 18 by turning on the light source 115, and causes the detection unit 114 to perform imaging in that state. Then, the processor 120 acquires the captured image from the detection unit 114, and cuts out the observation image 71 of the observation region 70 from the acquired captured image. Then, the processor 120 discriminates the change in the color development state of the color development region L3 of the observation image 71. In a case where it is discriminated that a change in the color development state of the color development region L3 is present, that is, in a case where the color of the color development region L3 has changed from a dark green color to an orange color, it means that the first reagent 41 has reached the color development region L3, and the assay region L1 and the control region L2 which are on the upstream side of the color development region L3.

In a case where the color development state of the color development region L3 has changed (Step S21: Yes), the processor 120 operates the second reagent supply mechanism 118 to start the supply of the second reagent 46 (Step S22). In the present embodiment, the processor 120 presses the second pressed part 12 of the cartridge 100 by the second reagent supply mechanism 118. In a case where the second pressed part 12 is pressed, the second pressed part 12 is deformed to sink toward the second reagent holding part 45. Due to this deformation, the sheet member 48 of the second reagent holding part 45 is pressed against the protrusion 34 to break, and the second reagent 46 is supplied onto the carrier 2.

On the other hand, in a case where the color development state of the color development region L3 has not changed (Step S21: No), the determination of whether or not it is within the fourth setting time t4 and whether or not the number of times n of discriminating the change in the color development state of the color development region L3 is less than K times (n < K) is performed (Step S23).

Here, in a case where the process has exceeded the fourth setting time t4 or it is not n < K (Step S23: No), the processor 120 notifies the error (Step S26) and ends the assay flow. For example, the notification of the error is performed by displaying an error message on the monitor 119. In addition to displaying the error message on the monitor 119, as a method of notifying the error, the error message may be notified by voice.

On the other hand, in a case where the it is within the fourth setting time t4 and it is n < K (Step S23: Yes), the process waits until the third setting time t3 elapses (Step S24). In Step S24 of Fig. 10, it is shown as "t3 wait". The third setting time t3 is, for example, about 30 seconds, and the fourth setting time t4 is preset to, for example, 20 minutes or the like. After that, n is incremented by 1 (indicated by n = n + 1 in Fig. 10) (Step S25), and the process returns to Step S21 of discriminating again whether or not the color development state of the color development region L3 has changed.

In Step S22, after the second reagent supply mechanism 118 is operated to start the supply of the second reagent 46, m = 1 is set in the processor 120 (Step S27). Here, m is a parameter of the number of times of executing the discrimination processing in the step of discriminating the presence or absence of bubbles (Step S27). Then, the processor 120 discriminates the presence or absence of bubbles in the assay region L1 (Step S28). In Step S28, specifically, similarly to Step S21, the processor 120 causes the detection unit 114 to image the observation region 70 in a state where the observation region 70 is illuminated. Then, the processor 120 cuts out an observation image 71 corresponding to the observation region 70 from the captured image output by the detection unit 114, and discriminates the presence or absence of bubbles based on the cut out observation image 71. A case where it is discriminated that bubbles is absent in the observation image 71 as in the observation image 71B (see Fig. 8B) means a state where the quantitativeness of the color development state of the assay region L1 is good, and whether the observation image 71 is positive or negative can be appropriately discriminated.

In a case where it is discriminated that bubbles is absent in the observation image 71 (Step S28: Yes), it is determined whether or not the line-shaped control region L2 is expressed (Step S32). In Step S32, the processor 120 discriminates the change in the color development state of the control region L2 from the observation image 71. The processor 120 discriminates, for example, whether or not the color optical density of the control region L2 reaches a density not less than a preset reference, and in a case where the density is not less than the reference, the processor 120 discriminates that the control region L2 is expressed. The case where it is discriminated that the control region L2 is expressed means that the sample 50 has reached the control region L2 and the assay region L1 on the upstream side thereof.

On the other hand, in a case where it is discriminated that bubbles is present in the observation image 71 (Step S28: No) as in the observation image 71A (see Fig. 8A), the determination of whether or not the it is within the second setting time t2 and whether or not the number of times m of discriminating the presence or absence of the bubbles is less than L times (m < L) is performed (Step S29).

Here, in a case where the process has exceeded the second setting time t2 or it is not m < L (Step S29: No), the processor 120 notifies the error (Step S26) and ends the assay flow.

On the other hand, in a case where the it is within the second setting time t2 and it is m < L (Step S29: Yes), the process waits until the first setting time t1 elapses (Step S30). In Step S30 of Fig. 10, it is shown as "t1 wait". The first setting time t1 is, for example, about 10 seconds, and the second setting time t2 is preset to, for example, 5 minutes or the like. After that, m is incremented by 1 (indicated by m = m + 1 in Fig. 10) (Step S31), and the process returns to Step S28 for discriminating again the presence or absence of bubbles.

In the discrimination of whether or not the control region L2 is expressed (Step S32), in a case where the control region L2 is expressed (Step S32: Yes), the processor 120 performs the main determination of whether the sample 50 is positive or negative (Step S33), and ends the assay flow.

For example, in the main determination (Step S33), the processor 120 discriminates whether the color optical density of the line-shaped assay region L1 reaches a density not less than a preset reference, and in a case where the density is not less than the reference, the processor 120 discriminates that the assay region L1 is expressed. The case where it is discriminated that the assay region L1 is expressed means that the sample 50 is positive, and the case where it is discriminated that the assay region L1 is not expressed means that the sample 50 is negative. In this way, in the main determination, the processor 120 determines whether the sample 50 is positive or negative depending on the presence or absence of expression of the assay region L1.

In a case where it is discriminated that expression in the assay region L1 is present, the processor 120 that has performed the main determination displays the assay result as "positive" on the monitor 119. In addition, in a case where it is discriminated that expression in the assay region L1 is absent, the processor 120 displays the assay result as "negative" on the monitor 119.

On the other hand, in the discrimination of whether or not the control region L2 is expressed in Step S32, in a case where the control region L2 is not expressed (Step S32: No), an error is notified (Step S26) and the assay flow ends. In a case where the control region L2 is not expressed after the development of the second reagent 46, there is a possibility that the sample 50 has not been spotted. The first assay flow is as described above.

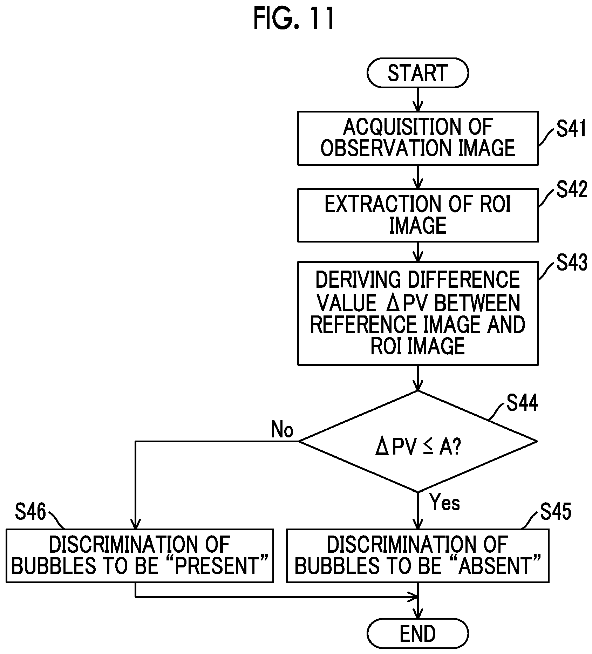

Here, the details of Step S28 of discriminating the presence or absence of bubbles in the first assay flow will be described. A plurality of flows can be considered as the flow of Step S28. Fig. 11 shows a first flow, which is an example of the flow of Step S28.

In the first flow shown in Fig. 11, the processor 120 first acquires the observation image 71 by cutting out the observation image 71 corresponding to the observation region 70 from the captured image output by the detection unit 114 (Step S41).

Next, the processor 120 extracts the ROI image 72 (see Fig. 8) of the region of interest (ROI) from the acquired observation image 71 (Step S42). In the present example, the region of interest ROI is a region that is the peripheral region of the assay region L1 and in which the line is not expressed (see Fig. 8). Then, the processor 120 reads out the reference image RI indicating the state where bubbles are absent and being held in the memory 121 as the setting information. The reference image RI may be an image held in the memory 121 in advance, or may be an image in a region where the line of the image acquired in Step S21 is not expressed. In Step S43, the processor 120 derives a difference value ΔPV, which is a difference of the pixel values between the reference image RI and the ROI image 72 extracted in Step S42. The ROI image 72 and the reference image RI each consist of a plurality of pixels two-dimensionally arranged in a matrix form. The difference value ΔPV is, as an example, a value obtained by calculating a difference of pixel values for each corresponding pixel of the ROI image 72 and the reference image RI and averaging differences in respective pixels.

In Step S44, the processor 120 determines whether or not the difference value ΔPV is not more than a preset threshold value A. In Step S44, in a case where the difference value ΔPV is not more than a preset threshold value A (ΔPV ≤ A) (Step S44: Yes), it is determined that bubbles are "absent" (Step S45), and the bubble discrimination step is ended. On the other hand, in a case where the difference value ΔPV is larger than the threshold value A (ΔPV > A) (Step S44: No), it is determined that bubbles are "present" (Step S46), and the bubble discrimination step is ended.

The region of interest ROI corresponding to the ROI image 72 is a region that is the periphery of the assay region L1 and in which the line is not expressed as described above. The region in which the line is not expressed shows the color of the carrier 2 itself that is the background of the assay region L1, and in a state where bubbles are absent, the density is substantially uniform. That is, the pixel values of the pixels included in the region are substantially uniform (see Fig. 8B). On the other hand, in a case where the bubbles are generated in the assay region L1, the bubbles are present also in the peripheral region thereof. Therefore, the pixel values of the pixels included in the region are not uniform, and the presence of bubbles causes shading. That is, since the image in which bubbles are present such as the ROI image 72A has shading, the pixel values vary, and the average value of the pixel values is smaller as compared with the image in which bubbles are absent such as the ROI image 72B (used for the reference image RI). Therefore, in a case where the difference value ΔRI from the reference image RI is not more than the threshold value A, it means that bubbles are absent or bubbles are present to the extent not to affect the discrimination of the line.

As described above, in the case of supplying the second reagent 46 to the carrier 2 in the cartridge 100, bubbles may be generated between the flow channel forming part 35 and the carrier 2, that is, on the assay region L1. In a case where the sample 50 is positive, the assay region L1 develops a black color, but in a case where bubbles are generated in the assay region L1, the visibility of the developed black color is significantly reduced by the bubbles. Therefore, in a case where the main determination (see Step S33 in Fig. 10) is performed in a state where bubbles are generated in the assay region L1, an erroneous determination may be made as negative even though the sample is positive. On the contrary, in a case where the sample 50 is negative, the assay region L1 does not develop a color, but in a case where bubbles are generated in the assay region L1, it is visually recognized such that the assay region L1 develops a color due to the increase of density as compared with a state where bubbles are absent, and thus an erroneous determination may be made as positive even though the sample is negative. However, in a case where the processor discriminates the bubbles to be absent based on the generation state of bubbles detected by the detection unit 114, the assay apparatus 110 of the present embodiment discriminates presence or absence of the change in the color development state of the assay region. Therefore, it is possible to suppress the occurrence of erroneous determination of the presence or absence of a change in the color development state due to the influence of bubbles, and it is possible to suppress the erroneous determination in the main determination of whether the sample is positive or negative.

In the above description, the processor 120 performs discrimination of the presence or absence of bubbles based on whether or not a difference (difference value ΔPV as an example) between the reference image RI in which bubbles are absent and the image of the region of interest ROI such as the ROI image 72 shown as an example is not more than a preset threshold value. The presence or absence of bubbles can be discriminated by a relatively simple method of comparing the images.

In the above-described first assay flow, the processor 120 performs discrimination of the presence or absence of bubbles based on the difference between the reference image RI and the ROI image 72, but may discriminate the presence or absence of bubbles by deriving the ratio of the reference image RI and the ROI image 72 and based on whether or not the ratio thereof is not more than a preset threshold value. The preset threshold values are different between the case where the difference is used and the case where the ratio is used.

As described above, in a case where the bubbles are discriminated to be present in the bubble discrimination step, after the discrimination, the processor 120 discriminates the presence or absence of bubbles again after a elapse of the preset first setting time t1. Accordingly, even in a case where the time from the generation of the bubbles to the disappearance of the bubbles varies due to individual differences of the cartridges, a reliable assay can be performed.

In addition, in a case where it is discriminated that the bubbles are present even after a lapse of a preset second setting time t2 from a preset time point after the cartridge 100 is loaded, or even after repeating the preset number of times of the discrimination, the processor 120 notifies an error. Accordingly, in a case where the bubbles do not disappear even after a elapse of the second setting time t2 or even after repeating the preset number of times of the discrimination, an error is notified, therefore, in a case where erroneous detection due to a defect in the assay apparatus 110, or the like occurs, it is possible to prevent the apparatus from being occupied indefinitely.

The preset time point at which the count of the second setting time t2 is started is appropriately set at the time when the supply of the second reagent 46 is started, at the time when the preset number of times of the discrimination of the presence or absence of bubbles is ended, or the like. In addition, the preset number of times L may be appropriately set to 2 or more.

In the first assay flow described above, in a case where discrimination of the presence or absence of bubbles is performed and the bubbles are discriminated to be present (Step S28: No), the determination of whether or not the it is within the second setting time t2 and whether or not the number of times m of discriminating the presence or absence of the bubbles is less than L times (m < L) (Step S29) is performed. Then, in a case where the it is within the second setting time t2 and it is m < L, the determination of the presence or absence of bubbles is repeated, and in a case where the process has exceeded the second setting time t2 or m has reached L, an error is notified (Step S26). However, in Step S29, only one of the conditions of within the second setting time t2 or less and m < L may be determined. That is, in Step S29, it is determined only whether or not the it is within the second setting time t2, and in a case where it is within the second setting time t2, the discrimination of the presence or absence of bubbles (Step S28) may be repeated, and in a case where it had exceeded the second setting time t2, an error may be notified (Step S26). In addition, in Step S29, it is determined only whether or not m < L, and in a case where m < L, the discrimination of the presence or absence of bubbles (Step S28) may be repeated, and in a case where m has reached L times, an error may be notified (Step S26).

In addition, in the first assay flow described above, in the case where it is discriminated that change in the color development state of the color development region L3 is absent, after the discrimination, the processor 120 discriminates the presence or absence of the change in the color development state again after a elapse of the preset third setting time t3. Accordingly, even in a case where the development time until the first reagent 41 reaches the color development region L3 varies due to individual differences of the cartridges 100, a reliable assay can be performed according to the development state of individual cartridge 100.

In addition, in the first assay flow, in a case where the color development state of the color development region L3 does not change even after a lapse of a preset fourth setting time t4 from a preset time point after the cartridge 100 is loaded, or even after repeating the preset number of times of the discrimination, the processor 120 notifies an error. Accordingly, in a case where the color development state does not change even after a elapse of the fourth setting time or even after repeating the preset number of times of the discrimination, an error is notified, therefore in a case of the defective development of the first reagent 41 or in a case where the first reagent 41 is not developed, it is possible to prevent the apparatus from being occupied by the cartridge 100 indefinitely.

In addition, the preset time point at which the count of the fourth setting time t4 is started may be the time when the cartridge is loaded, or the time when the preset number of times of the discrimination is ended. In addition, the preset number of times K may be appropriately set to 2 or more.

Also in Step S23, only one of the conditions of within the fourth setting time t4 or less and n < K may be determined. That is, in Step S23, it is determined only whether or not the it is within the fourth setting time t4, and in a case where it is within the fourth setting time t4, the discrimination of the presence or absence of the change in the color development state of the color development region L3 (Step S21) may be repeated, and in a case where it had exceeded the fourth setting time t4, an error may be notified (Step S26). In addition, in Step S23, it is determined only whether or not n < K, and in a case where n < K, the discrimination of the presence or absence of the change in the color development state of the color development region L3 (Step S21) may be repeated, and in a case where n has reached K times, an error may be notified (Step S26).

In the present embodiment, the detection unit 114 detects all the color development states of the assay region L1, the control region L2, and the color development region L3, and detects the generation state of bubbles generated in the assay region L1 due to the supply of the reagent, but may individually includes a detection unit (first detection unit) that detects the color development state and a detection unit (second detection unit) that detects the generation state of bubbles. However, as in the present embodiment, by using one detection unit 114 commonly, the configuration can be simplified as compared with the case where the detection units are individually provided, and the cost can be suppressed. In addition, since only one detection unit 114 is required, space can be saved.

The detection unit that detects the generation state of bubbles is not limited to an imaging unit that images an image, such as an image sensor, and may be a photodetector that can detect the light amount. Since the light amount of reflected light varies depending on the generation state of bubbles, the generation state of bubbles may be detected by detecting the change in the light amount with a photodetector.

In the present disclosure, an actuator such as a solenoid is exemplified as the second reagent supply mechanism 118, but a mechanism capable of starting the supply of the second reagent 46 from the second reagent holding part 45 according to the configuration of the second reagent holding part in the cartridge 100 may be used. For example, in a case where the second reagent holding part 45 includes a shutter and the supply of the second reagent 46 is started by opening the shutter, the second reagent supply mechanism 118 may be a mechanism for opening the shutter. The same applies to the first reagent supply mechanism 116.

In the above-described embodiment, the first reagent 41 is the first amplifying liquid and the second reagent 46 is the second amplifying liquid, but the first reagent 41 and the second reagent 46 are not limited to this combination. A combination in which the first reagent 41 is a developing solution and the second reagent 46 is a cleaning liquid, or a combination in which the first reagent 41 is a developing solution or a cleaning liquid and the second reagent 46 is an amplifying liquid may be used.

However, the second reagent 46 is preferably an amplification agent that amplifies color development. In a case where the second reagent 46 is an amplification agent that amplifies the color development of the assay region L1, the color development of the assay region L1 is amplified, and thus the determination accuracy can be improved.

In addition, as in the present embodiment, the first reagent 41 and the second reagent 46 are preferably the amplifying liquids that amplify the color development of the assay region L1. In a case where the first reagent 41 and the second reagent 46 is an amplification agent that amplifies the color development of the assay region L1, the color development of the assay region L1 is amplified, and thus the determination accuracy can be improved.

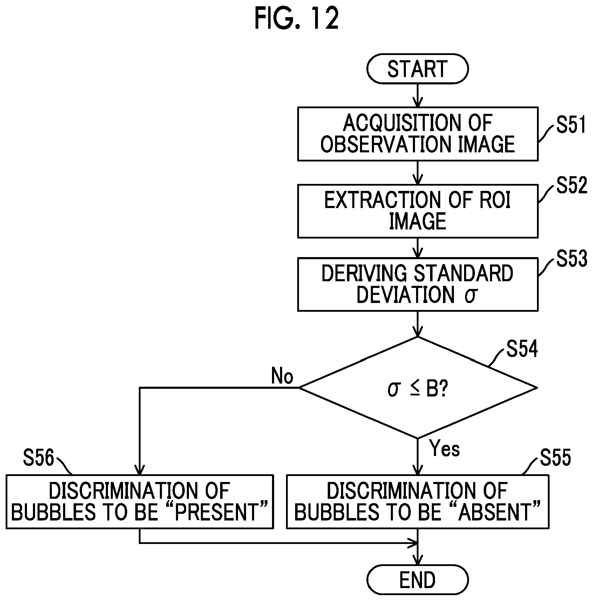

Step S28 (see Fig. 10) of discriminating the presence or absence of bubbles is not limited to the first flow described above. Fig. 12 shows a second flow which is another example of Step S28.

As shown in Fig. 12, in the second flow, the processor 120 acquires the observation image 71 by causing the detection unit 114 to image the observation region 70 in a state where the observation region 70 is illuminated by the light source 115 (Step S51).

Next, the processor 120 extracts the ROI image 72 (see Fig. 8) corresponding to the region of interest ROI from the acquired observation image 71 (Step S52). Similarly to the first flow, the region of interest ROI is a region in which the line of the peripheral region of the assay region L1 is not expressed.

In Step S53, the processor 120 derives the standard deviation σ of the pixel value of the ROI image 72 from respective pixel values of the ROI image 72. In a case where the derived standard deviation σ is not more than a preset threshold value B (that is, σ ≤ B) (Step S54: Yes), the processor 120 discriminates that bubbles are "absent" (Step S55), and ends the bubble discrimination step. On the other hand, in a case where the standard deviation σ is larger than the threshold value B (σ > B) (Step S54: No), the processor 120 discriminates that the bubbles are "present" (Step S56), and ends the bubble discrimination step.

The region of interest ROI is, as described above, for example, a region that is the periphery of the assay region L1 and in which the line is not expressed. Since the region where the line is not expressed shows the color of the carrier 2 itself, the density is substantially uniform in a state where there are no bubbles. That is, the pixel values of the pixels included in the region are substantially uniform, and thus the standard deviation σ is small. On the other hand, in a case where the bubbles are generated in the assay region L1, the bubbles are present also in the peripheral region thereof. Therefore, the pixel values of the pixels included in the region are not uniform, and the presence of bubbles causes shading. Since the image in which bubbles are present has shading, the pixel values vary, and the standard deviation σ is large. That is, in a case where the standard deviation σ is not more than the threshold value B, it means that bubbles are absent or bubbles are present to the extent not to affect the discrimination of the line.

In the second flow, which is another example of Step S28 of discriminating the presence or absence of bubbles, the processor 120 derives a standard deviation σ of a pixel value of the acquired image of the region of interest ROI (as an example, the ROI image 72), and discriminates the bubbles to be absent in the case where the standard deviation σ is not more than the threshold value B, and therefore, it is possible to discriminate the presence or absence of the generation of bubbles without comparing with the image in which bubbles are absent, such as the reference image RI, as in the first flow.

### (Modified example)

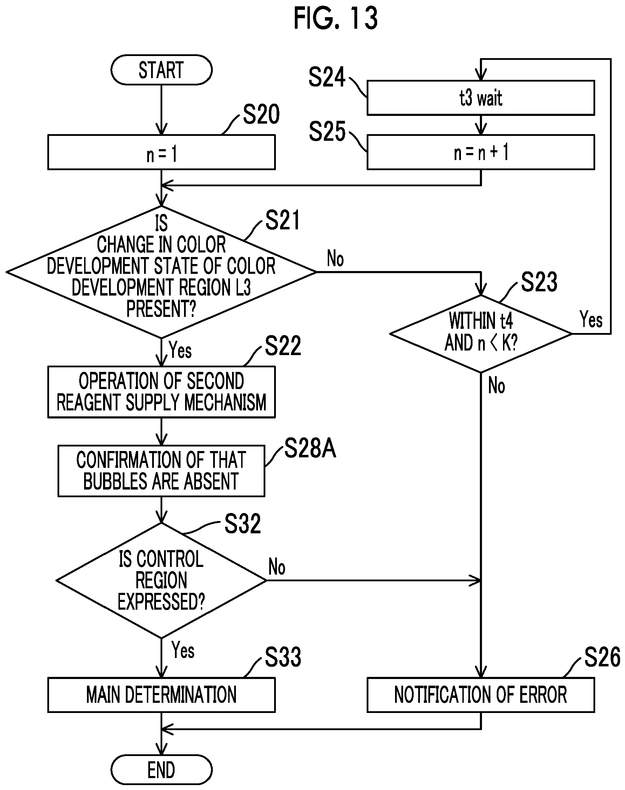

Instead of Step S28 of discriminating the presence or absence of bubbles in the first assay flow, as shown in Fig. 13, Step S28A of detecting a change with time in a generation state of bubbles and confirming that the bubbles have disappeared may be included. Fig. 13 is an assay flow of a modified example in which the first assay flow in the assay apparatus 110 shown in Fig. 10 is partially modified. In Fig. 13, the same steps as those of the first assay flow in Fig. 10 are denoted by the same reference numeral for step, and detailed description thereof will be omitted.

In the assay flow shown in Fig. 13, it is confirmed that the bubbles have disappeared after Step S22 of operating the second reagent supply mechanism 118 to supply the second reagent 46 (Step S28A). After confirming that the bubbles have disappeared, it is discriminated whether or not the line-shaped control region L2 is expressed (Step S32). Other steps are the same as the assay flow shown in Fig. 10.

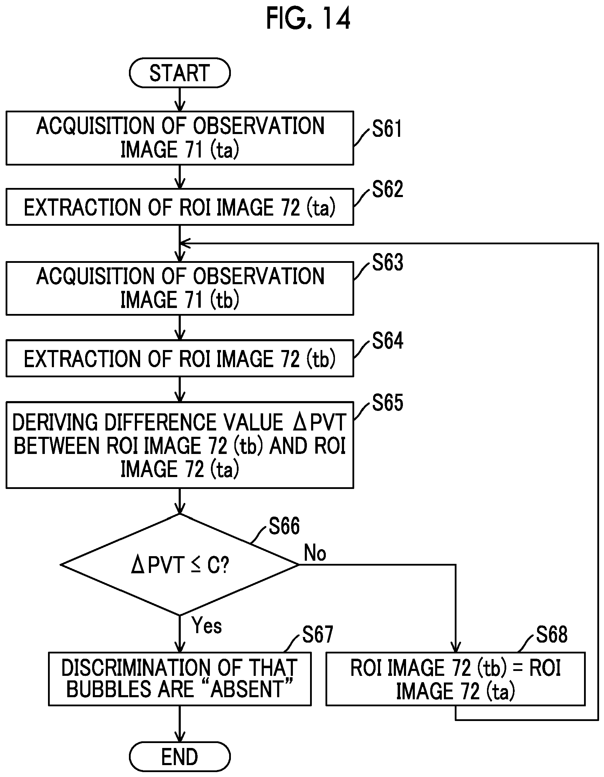

Fig. 14 is a diagram showing a processing flow in Step S28A of confirming that the bubbles have disappeared.

The processor 120 acquires the observation image 71 (ta) obtained by imaging the observation region 70 at the time ta, through the detection unit 114 (Step S61). Then, the ROI image 72 (ta) of the region of interest ROI is extracted from the observation image 71 (ta) (Step S62). In addition, the processor 120 acquires the observation image 71 (tb) obtained by imaging the same observation region 70 at time tb (Step S63), and extracts the ROI image 72 (tb) of the region of interest ROI from the observation image 71 (tb) (Step S64). Similarly to the above, the region of interest ROI is, for example, a peripheral region of the assay region L1 and is a region in which the line is not expressed.

Next, the processor 120 derives a difference value ΔPVT of the pixel values between the ROI image 72 (ta) extracted from the observation image 71 (ta) and the ROI image 72 (tb) extracted from the observation image 71 (tb) (Step S65). Then, it is discriminated whether or not the difference value ΔPVT is not more than the preset threshold value C (Step S66).

In a case where the difference ΔPVT is larger than the threshold value C (Step S66: No), the ROI image 72 (tb) is defined as a new ROI image 72 (ta) (Step S68), the process returns to Step S63 of acquiring the new observation image 71 (tb), and Step S63 to Step S66 are repeated until bubbles are disappeared.

As time elapses from the initial state in which bubbles are generated, the bubbles move or disappear, and thus the amount of bubbles present in the assay region L1 gradually reduced. In the initial stage in which the amount of bubbles in the assay region L1 is large, the reduction amount of bubbles per unit time is large, and as time elapses, the reduction amount of in bubbles per unit time decreases. Eventually, the bubbles are reduced to such an extent that the main determination of detecting the change in the color development state of the assay region L1 is not affected. That is, the reduction rate of the bubbles is large at the initial stage of starting to reduce, gradually decreases, and eventually, when the reduction rate becomes not more than the certain amount, the bubbles are reduced to an extent that the main determination is not affected.

The difference value ΔPVT means a reduction amount of bubbles per unit time, and a difference value ΔPVT larger than the threshold value C means a large reduction amount of bubbles due to movement or disappearance of the bubbles. The fact that the reduction amount of bubbles is large means that the state in which the bubbles are significantly reduced continues, and means that a certain amount of bubbles is still present in the assay region L1. On the other hand, the fact that the difference value ΔPVT is not more than the threshold value C means that the change in the amount of the bubbles due to the movement or disappearance of the bubbles is small, and the bubbles are reduced to such an extent that the main determination is not affected.

In a case where the difference value ΔPVT is not more than the preset threshold value C (Step S66: Yes), the processor 120 discriminates that the bubbles are "absent" (Step S67), and ends Step S28A of confirming that the bubbles have disappeared.

In this way, the processor 120 acquires two or more observation images having a time difference through the detection unit 114, and derives the difference of the pixel values of the two or more images of the region of interest ROI (as an example, the ROI image 72), in a case where the derived difference is not more than a preset threshold value, the bubbles are discriminated to be absent, and therefore the change with time in a generation state of bubbles indicating that the bubbles are disappeared from the state in which the bubbles are generated can be discriminated by a simple method of comparing images having a time difference.

In the above description, the processor 120 discriminates the presence or absence of bubbles based on whether or not the difference of the pixel values of the two ROI images 72 is more than a preset threshold value. However, the processor 120 may discriminate the presence or absence of bubbles by deriving the ratio of the pixel values of the two ROI images 72 and based on whether or not the ratio thereof is not more than a preset threshold value. The preset threshold values are different between the case where the difference is used and the case where the ratio is used.

In the above-described embodiment, the case of the first assay flow in which the supply of the first reagent 41 is performed by the user and the supply of the second reagent 46 is performed in the assay apparatus 110 has been described. As described above, in the assay apparatus 110, the second assay flow and the third assay flow can also be selected.

In the second assay flow, after the user spots the sample 50, the cartridge 100 is loaded into the loading part 112 of the assay apparatus 110 without supplying the first reagent 41. In the assay apparatus 110 in which the cartridge 100 is loaded, the processor 120 first operates the first reagent supply mechanism 116 to supply the first reagent 41 to the carrier 2. Subsequent processing is the same as that of the first assay flow descried above.

In the third assay flow, the user spots the sample 50 and supplies the first reagent 41, and after a lapse of a predetermined time, the user supplies the second reagent 46, and then the cartridge 100 is loaded into the loading part 112 of the assay apparatus 110. In the assay apparatus 110 in which the cartridge 100 is loaded, the processor 120 performs a step of discriminating the presence or absence of bubbles or a step of confirming that the bubbles have disappeared. Subsequent processing is the same as that of the first assay flow descried above.

In the above-described embodiment, as the processor 120 and a hardware structure of a processing unit as internal configurations thereof that executes various types of processing, such as a detection unit control unit 122, a color development state discrimination unit 123, a first reagent supply mechanism control unit 124, a second reagent supply mechanism control unit 125, a display control unit 126, and a bubble discrimination unit 129, various processors shown below can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors, or may be configured by a combination of two or more processors having the same type or different types (for example, a combination of a plurality of FPGAs and/or a combination of a CPU and an FPGA). In addition, a plurality of processing units may be formed of one processor.

As an example in which a plurality of processing units are configured into a single processor, there is a form in which a single processor is configured by a combination of one or more CPUs and software, and this processor functions as a plurality of processing units. A second example of the configuration is an aspect in which a processor that implements the functions of the entire system including a plurality of processing units using one integrated circuit (IC) chip is used. A representative example of this aspect is a system on chip (SoC). In this way, various processing units are configured by one or more of the above-described various processors as hardware structures.

Furthermore, specifically, an electric circuit (circuitry) obtained by combining circuit elements, such as semiconductor elements, can be used as the hardware structure of the various processors.

The disclosure of Japanese patent application 2021-050778 filed on March 24, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. An immunochromatographic assay apparatus comprising:
a loading part in which a cartridge that includes a carrier having a spotting region on which a sample is spotted and an assay region in which a color development state changes depending on whether the sample is positive or negative, and in which a reagent is supplied to the carrier, is attachably and detachably loaded;
a first detection unit that detects a color development state in the assay region;
a second detection unit that detects a generation state of bubbles generated in the assay region due to the supply of the reagent; and
a processor configured to discriminate presence or absence of a change in the color development state based on the color development state detected by the first detection unit, and discriminate the presence or absence of the change in the color development state in a case where the bubbles are discriminated to be absent based on the generation state of the bubbles detected by the second detection unit.

2. The immunochromatographic assay apparatus according to claim 1,
wherein the second detection unit is an image sensor that images an observation region including the assay region and a peripheral region of the assay region to output an observation image including the observation region.

3. The immunochromatographic assay apparatus according to claim 2,
wherein the processor is configured to obtain a standard deviation of pixel values of an image of a region of interest within the observation region, and discriminate the bubbles to be absent in a case where the standard deviation is not more than a preset threshold value.

4. The immunochromatographic assay apparatus according to claim 2,
wherein the processor is configured to derive a difference or a ratio between a pixel value, between a reference image in a state where bubbles are absent and an image of a region of interest within the observation region, and discriminate the bubbles to be absent in a case where the difference or the ratio is not more than a preset threshold value.

5. The immunochromatographic assay apparatus according to any one of claims 1 to 4,
wherein in a case where the bubbles are discriminated to be present, the processor is configured to discriminate presence or absence of the bubbles again after a lapse of a preset first setting time.

6. The immunochromatographic assay apparatus according to any one of claims 1 to 5,
wherein in a case where it is discriminated that the bubbles are present even after a lapse of a preset second setting time from a preset time point after the cartridge is loaded, the processor is configured to notify an error.

7. The immunochromatographic assay apparatus according to claim 2,
wherein the processor is configured to acquire two or more of observation images having a time difference from the second detection unit, derive a difference or a ratio between pixel values, between images of a region of interest within the observation region in the two or more of observation images, and discriminate the bubbles to be absent in a case where the difference or the ratio is not more than a preset threshold value.

8. The immunochromatographic assay apparatus according to any one of claims 1 to 7,
wherein the first detection unit is an imaging unit that images an observation region including the assay region and a peripheral region thereof, and the first detection unit and the second detection unit are used in common.

9. The immunochromatographic assay apparatus according to any one of claims 1 to 8,
wherein the cartridge includes a cover member that covers the carrier, and in the cover member, has a gap of 0.01 to 1 mm in which a reagent is supplied between a surface of the carrier in the assay region and the cover member, and the reagent is developed in the assay region using the gap as a flow channel.

10. The immunochromatographic assay apparatus according to any one of claims 1 to 9,
wherein the cartridge includes a first reagent holding part that holds a first reagent to be supplied to the carrier and a second reagent holding part that holds a second reagent to be supplied to the carrier after the first reagent is supplied to the carrier,
the reagent that is a cause for generation of the bubbles is the second reagent, and
the processor is configured to discriminate presence or absence of the bubbles after the second reagent is supplied to the carrier.
